# EUROPEAN PATENT APPLICATION

(11) **EP 4 609 846 A1**
(43) Date of publication of application: **03.09.2025**
(21) Application number: 23882963.4
(22) Date of filing: 12.10.2023
(51) Int. Cl.: A61K 8/02, A61Q 1/00, A45D 34/00

(54) **COSMETIC-IMPREGNATED SPONGE AND COSMETIC PRODUCT COMPRISING SAME**

(30) Priority: 28.10.2022 KR 20220141850
(71) Applicant: Cosmax, Inc., Hwaseong-si, Gyeonggi-do 18622 (KR)
(72) Inventor: YOO, Eun Kyung, Seongnam-si, Gyeonggi-do 13590 (KR); AHN, You Jin, Yongin-si, Gyeonggi-do 17003 (KR); LEE, Wha Young, Yongin-si, Gyeonggi-do 16903 (KR); SEO, Eun Ju, Suwon-si, Gyeonggi-do 16433 (KR); PARK, Myeong Sam, Seoul 04987 (KR)
(74) Representative: Mammel und Maser Patentanwälte PartG mbB
(86) International application number: PCT/KR2023/015733
(87) International publication number: WO 2024/090856

(57) **Abstract**

The present invention relates to: a sponge foam for cosmetic material impregnation comprising a sponge for cosmetic material impregnation, and a cosmetic product comprising same. The present invention provides the cosmetic-impregnated cosmetic product comprising: a first block including an impregnation sponge to be impregnated with a liquid cosmetic material; and a second block stacked on the first block, wherein the second block includes an incision pattern formed by a slit penetrating the second block in a thickness direction.

## Description

### Technical Field

The present invention relates to a sponge for cosmetic material impregnation and, more specifically, to a sponge foam for cosmetic material impregnation including a cosmetic impregnation sponge, a cosmetic article including the same, and a manufacturing method therefor.

### Background Art

Cosmetic articles encompassing a sponge-type impregnation member in which a cosmetic composition is loaded within open pores dispersed internally are widely used. Such cosmetic articles with sponge can easily release a cosmetic material as a content through mechanical compression, and the released cosmetic material may be applied to the skin by being coated onto a cosmetic tool such as a puff.

However, an attempt has been made to engrave a certain pattern on the surface of a sponge to enhance the beauty of the sponge due to its dull appearance and facilitate the release characteristics of a cosmetic material. For example, Korean Patent No. 10-1566803 discloses that the surface of a sponge is compressed by a heated mold with a recess and protrusion pattern to obtain an inverse pattern, thereby promoting the uniform release of contents.

However, such a recess and protrusion pattern formed in a conventional heating manner collapses open-cell or net-shape pore structures and cell structures since the surface of the sponge is inevitably melted or deformed during compression, and consequently, achieving smooth release of contents becomes difficult when a puff is compressed.

Meanwhile, a laser marking method of forming a surface pattern through a laser beam in order to minimize the deformation of a pore structure for facilitating the release of a cosmetic material has been proposed in order to solve the problem, but the deformation still occurs in a processing area due to laser heat and the processing time required for laser marking is long.

### Disclosure of Invention

### Technical Problem

An aspect of the present invention is to provide a sponge for cosmetic material impregnation having a surface marking pattern.

Another aspect of the present invention is to provide a sponge for cosmetic material impregnation that suffers no substantial melting or deformation.

Still another aspect of the present invention is to provide a sponge for cosmetic material impregnation that suffers no substantial deformation of its pore structure.

Still another aspect of the present invention is to provide a sponge for cosmetic material impregnation having a surface marking pattern with a structure that does not interrupt the release of a cosmetic material.

Still another aspect of the present invention is to provide a cosmetic article including the above-described sponge for cosmetic material impregnation.

### Solution to Problem

In accordance with an aspect of the present invention, there is provided a cosmetic article for cosmetic material impregnation, including: a first block including an impregnation sponge impregnated with a liquid cosmetic material; and a second block stacked on the first block, wherein the second block includes an incision pattern formed by a slit penetrating the second block in a thickness direction.

The cosmetic article may further include an adhesive layer binding the first block and the second block. The adhesive layer may be formed at the outside of the incision pattern of the second block.

The adhesive layer may be formed along the outer periphery of the second block, and for example, the adhesive layer may have a ring shape.

The adhesive layer may contain a water-soluble adhesive, and for example, the adhesive may be a polyurethane resin.

The cosmetic article may further include a third block between the first block and the second block.

The slit for the incision pattern may be formed by a knife blade.

The first block may include a polyurethane foam. In addition, the second block may include a polyurethane foam or a fabric or non-woven fabric.

The first block may have a number of pores per inch (ppi) of 30 to 200.

In accordance with another aspect of the present invention, there is provided a stacked sponge for cosmetic material impregnation, including: a first block including a liquid cosmetic material impregnation sponge; a second block stacked on the first block; and an adhesive layer disposed between the first block and the second block, wherein the second block includes an incision pattern formed by a slit penetrating the second block in a thickness direction.

The adhesive layer may be formed at the outside of the incision pattern of the second block.

### Advantageous Effects of Invention

According to the present invention, a sponge for cosmetic material impregnation can be provided that suffers no substantial deformation of its porous structure during the formation of a surface pattern.

Furthermore, according to the present invention, the sponge for cosmetic material impregnation suffers no substantial melting or deformation even during the formation of the surface pattern.

Furthermore, according to the present invention, a sponge for cosmetic material impregnation can be provided that suffers no substantial deformation of its porous structure and has a surface pattern with a structure that does not interrupt the release of a cosmetic material.

### Brief Description of Drawings

FIG. 1 schematically shows a cosmetic article according to an embodiment of the present invention.
FIG. 2 is an exploded perspective diagram schematically showing the appearance of a stacked impregnation member according to an embodiment of the present invention.
FIG. 3 is a diagram schematically showing an example of a cross section of the stacked impregnation member of FIG. 1.
FIGS. 4A and 4B are diagrams showing an example of a cosmetic article including an impregnation member according to an embodiment of the present invention.
FIG. 5 is a photograph image of the configuration of a test device.

### Best Mode for Carrying out the Invention

Hereinafter, preferable embodiments of the present invention will be described with respect to the drawings.

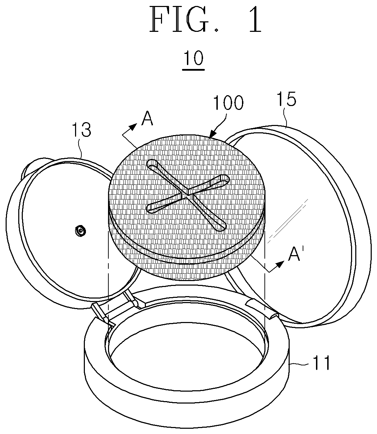

FIG. 1 schematically shows a cosmetic article according to an embodiment of the present invention.

As shown in the drawing, a cosmetic article 10 includes a case body 11 for accommodating a liquid cosmetic material. The case body 11 accommodates a stacked impregnation member 100 impregnated with a liquid cosmetic material. The cosmetic article may include an inner lid 13 and/or an outer lid 15 for preventing the leakage of the liquid cosmetic material from the stacked impregnation member 100. Although not shown, a fixing element for fixing the impregnation member may be further provided in order to prevent the detachment of the impregnation member. This will be described separately later.

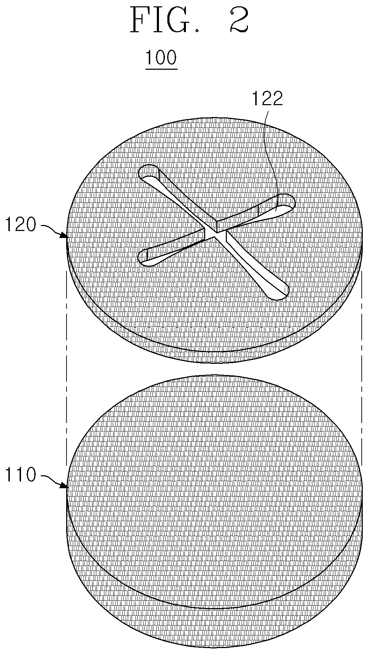

FIG. 2 is an exploded perspective diagram schematically showing the appearance of a stacked impregnation member according to an embodiment of the present invention.

Referring to FIG. 2, the stacked impregnating member 100 is composed of a stacked body of a first block 110 and a second block 120.

In the present invention, the first block 110 may be formed of a foamed synthetic resin. Foam pores inside the first block 110 are connected to each other to form open channels. The pore structure of the first block 110 may be an open-cell foam or a network-type foam structure. The network-type foam refers to a foam in which the sponge has a network-type structure with cell walls that are open. For example, in the present invention, the number of pores per inch (ppi) in the sponge 110 may be 30 to 200 or 70 to 200.

In the present invention, various synthetic resins may be used as materials for the first block 110, and for example, a polyether, a polyester, a polyurethane, polyvinyl alcohol (PVA), polyethylene (PE), ethylene vinyl acetate (EVA), acrylonitrile butadiene styrene (ABS), or a synthetic rubber may be used.

Illustratively, the polyurethane may be a polyurethane prepared using a polyether polyol, a polyester polyol, a mixture thereof, or a copolymer thereof as a raw material, and the synthetic rubber may be at least one type of rubber selected from the group consisting of styrene-butadiene rubber (SBR), butadiene rubber (BR), natural rubber (NR), and acrylonitrile-butadiene rubber (NBR).

Meanwhile, the second block 120 is stacked on the first block 110. The second block 120 may be implemented using a foamed synthetic resin as described above, but is not limited thereto, and thus the second block may also be implemented using a fabric or a non-woven fabric.

The second block 120 has an incision pattern 122 forming a surface pattern of the stacked impregnation member. In the present invention, the incision pattern 122 may be implemented by extending a through slit, penetrating the first block, in a thickness direction. That is, in the present invention, the incision pattern may correspond to a closed curve area defined by the through slit. When the processed block piece within the closed curve area formed by the through slit is removed, the remaining portion integrates into one body without separation.

This through slit may be easily formed using a non-heating type machine tool, such as a knife blade, and no damage or deformation of the material of the block occurs during the formation of the through slit.

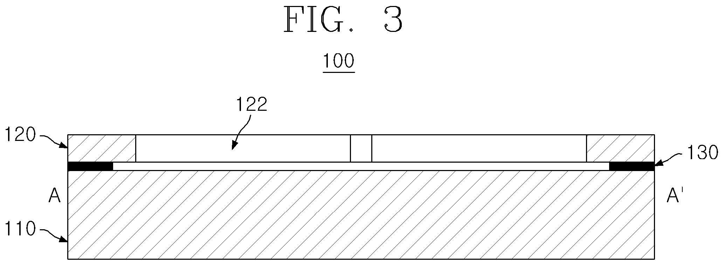

FIG. 3 is a diagram schematically showing an example of a cross section of the stacked impregnation member of FIG. 1.

Referring to FIG. 3, an adhesive layer 130 is formed between the first block 110 and the second block 120.

In the present invention, the adhesive layer 130 may employ various materials. In the present invention, an epoxy resin, an acrylic resin, a polyurethane resin, a synthetic rubber adhesive, or the like may be used as an adhesive for forming the adhesive layer 130. Preferably, a water-soluble polyurethane resin may be used as the adhesive.

In an embodiment of the present invention, the adhesive layer 130 may be formed at the outside of the incision pattern of the second block, on one surface of at least one of the first block 110 and the second block 120. In an embodiment, the adhesive layer 130 may be formed on one surface of the first block 110, at the site corresponding to the outside of the incision pattern of the second block. In another embodiment, the adhesive layer 130 may be formed on one surface of the second block 120, at the site corresponding to the outside of the incision pattern of the second block. In still another embodiment, the adhesive layer 130 may be formed on one surface of the first block 110 and one surface of the second block 120, at the site corresponding to the outside of the incision pattern of the second block.

In the present invention, the adhesive layer 130 is preferably restricted to the outside of the slit pattern (outside of the slit closed curve area) of the second block. Such a placement of the adhesive layer allows the movement of the liquid cosmetic composition through the incision pattern, thereby facilitating the movement of the cosmetic material from the first block to the second block or vice versa. Consequently, this can facilitate the filling of the cosmetic composition into the stacked impregnation member and the release of the cosmetic composition from the stacked impregnation member.

When the incision pattern 122 is placed in the center portion of the second block 120, and the adhesive layer is disposed outside the incision pattern, the following advantages can be obtained. As described above, in the present invention, the inner wall of the incision pattern is formed without damage or deformation, so that the pore structure holds its original open pore structure as it **is.** Therefore, when a user compresses the center of the upper surface of the stacked impregnation member 100 by using a tool such as a puff, the liquid cosmetic material impregnated inside the first block can be easily transferred to the second block through the incision pattern at the center. Therefore, a structure in which a cosmetic material can be continuously released despite the presence of adhesive can be attained.

More specifically, the adhesive layer 130 may be disposed along the outer periphery of the second block. For example, the adhesive layer 130 may have a ring shape that is extended along the outer periphery of the second block at the outside of the incision pattern of the second block. This structure enables the adhesive to move through a non-adhesive region in the area outside the incision pattern of the second block.

Hereinabove, the first block 110 and the second block 120 having the same two-dimensional shapes defined by the same width and depth have been described, but the present invention is not limited thereto. Since the blocks constituting the stacked structure are separate and distinct from each other, the blocks may have different sizes. For example, the second block 120 may have a smaller width or depth than the first block 110, and vice versa.

Although the stacked impregnation member composed of two blocks has been described with reference to FIGS. 2 and 3, the present invention is of course not limited thereto, and one or more intermediate blocks may be interposed between the first and second blocks or may be provided below the second block. The additional blocks may be implemented using a foamed synthetic resin, a fabric, a non-woven fabric, or the like.

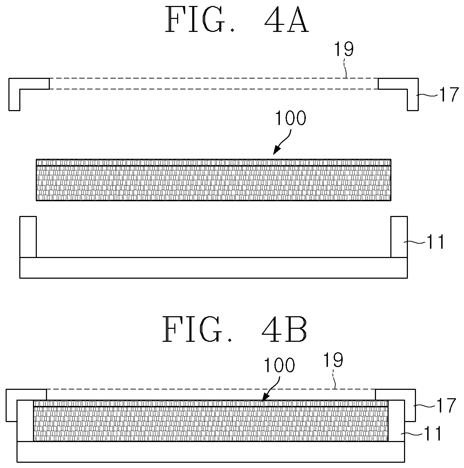

FIGS. 4A and 4B are diagrams showing an example of a cosmetic article including an impregnation member according to an embodiment of the present invention. FIG. 4A is a schematic diagram of an exploded state of a cosmetic article, and FIG. 4B is a schematic diagram of an assembled state of the cosmetic article.

Referring to FIG. **4****,** the stacked impregnation member 100 is accommodated in the container body 11 for accommodating an impregnation member. An impregnation member fixture 17 having an opening 19 in the center is mounted on the stacked impregnation member 100, thereby preventing the detachment or separation of the stacked impregnation member.

### Mode for Carrying out the Invention

### <Test Example>

A test was conducted to measure the release characteristics of a stacked impregnation member prepared without an adhesive and a stacked impregnation member prepared by the application of an adhesive.

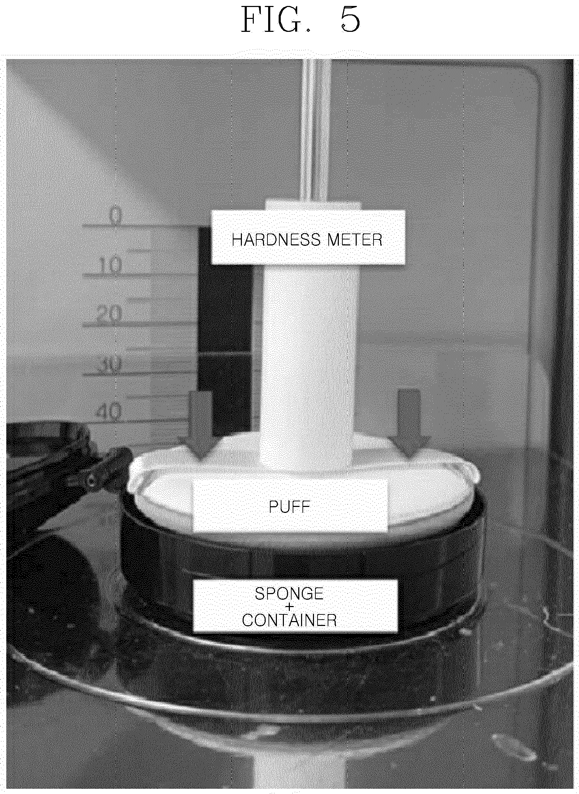

A stacked impregnation member species prepared by stacking two sheets of polyurethane sponges without an adhesive and a stacked impregnation member species manufactured by laminating two sheets of polyurethane sponges with an adhesive were prepared. The adhesive was applied along the vicinity of the edge of the sponge. Each impregnation member specimen with a cosmetic material deposited therein was accommodated in a container, and then a puff was placed on the sponge, and subsequently, the same force was used to press the sponge by using a hardness meter. A comparison was made on the release amount of each stacked impregnation member by comparing the amounts of content collected by the puff. FIG. 5 is a photograph image of the configuration of a test device. Particularly, almost the same amount of content was deposited in each impregnation member specimen.

**TABLE 1**

| Classification | Filling amount (g) | Puff weight (g) | | Release amount (g)/ Release ratio |
|---|---|---|---|---|
| Impregnation member prepared by staking with adhesive | 12.1 | Before | 1.64 | 0.42/3.47% |
| | | After | 2.06 | |
| Impregnated member prepared by staking without adhesive | 12.02 | Before | 1.67 | 0.34/2.82% |
| | | After | 2.01 | |

Table 1 above identified that the impregnation member prepared by staking using an adhesive showed a release amount of content similar to that of the impregnation member prepared by stacking without an adhesive.

The stacking of the first and second blocks and the binding thereof using an adhesive have been described above, but the stacked body of the present invention may be implemented in other manners. For example, the binding may be implemented by compressing the vicinity of the edge of the stacked impregnation member through ultrasonic heating or the like to form an edge portion. Particularly, the stacked impregnation member may have a lens shape that is convex upwards and downwards.

While the present invention has been described above with reference to the exemplary embodiments of the present invention, the above-mentioned description is provided just for illustrating the present invention, and the present invention is not limited thereto. It should be considered by those skilled in the art that various modifications may be made without departing from the appended claims and the subject matter of the present invention and these modifications fall into the scope of the present invention.

### Industrial Applicability

The present invention is applicable to a cosmetic impregnation sponge and a cosmetic article.

## Claims

1. A cosmetic article for cosmetic material impregnation, comprising:
a first block comprising an impregnation sponge impregnated with a liquid cosmetic material; and
a second block stacked on the first block,
wherein the second block comprises an incision pattern formed by a slit penetrating the second block in a thickness direction.

2. The cosmetic article of claim 1, further comprising an adhesive layer binding the first block and the second block.

3. The cosmetic article of claim 2, wherein the adhesive layer is formed at the outside of the incision pattern of the second block.

4. The cosmetic article of claim 2, wherein the adhesive layer is formed along the outer periphery of the second block.

5. The cosmetic article of claim 4, wherein the adhesive layer has a ring shape.

6. The cosmetic article of claim 1, wherein the adhesive layer contains a water-soluble adhesive.

7. The cosmetic article of claim 6, wherein the adhesive is a polyurethane resin.

8. The cosmetic article of claim 1, further comprising a third block between the first block and the second block.

9. The cosmetic article of claim 1, wherein the slit for the incision pattern is formed by a knife blade.

10. The cosmetic article of claim 1, wherein the first block comprises a polyurethane foam.

11. The cosmetic article of claim 1, wherein the second block comprises a polyurethane foam.

12. The cosmetic article of claim 1, wherein the second block comprises a fabric or non-woven fabric.

13. The cosmetic article of claim 1, wherein the first block has a number of pores per inch (ppi) of 30 to 200.

14. A stacked sponge for cosmetic material impregnation, comprising: a first block comprising a liquid cosmetic material impregnation sponge; a second block stacked on the first block; and an adhesive layer disposed between the first block and the second block,
wherein the second block comprises an incision pattern formed by a slit penetrating the second block in a thickness direction.

15. The stacked sponge for cosmetic material impregnation of claim 14, wherein the adhesive layer is formed at the outside of the incision pattern of the second block.
